# EUROPÄISCHE PATENTSCHRIFT

(11) **EP 1 459 780 B1**
(45) Veröffentlichungstag und Bekanntmachung des Hinweises auf die Patenterteilung: **07.06.2006**
(21) Anmeldenummer: 03006470.3
(22) Anmeldetag: 21.03.2003
(51) Int. Cl.: A61M 25/01

(54) **Platziervorrichtung für einen Katheter**
Placement device for a catheter
Dispositif de placement pour un cathéter

(43) Veröffentlichungstag der Anmeldung: 22.09.2004
(73) Patentinhaber: Urovision Gesellschaft für Medizinischen Technologie Transfer mbH, 83043 Bad Aibling (DE)
(72) Erfinder: Jentsch, Cordula, 14943 Luckenwalde (DE)
(74) Vertreter: Hofstetter, Alfons J.

(56) Entgegenhaltungen:
- WO-A-01/60433
- DE-U- 20 113 815
- US-A- 5 176 647

## Beschreibung

Die vorliegende Erfindung betrifft eine Platziervorrichtung für einen Katheter zum Einführen des Katheters in Körperöffnungen und Körpergefäße bestehend aus einer an einem Ende des Katheters lösbar mit dem Katheter verbundenen, länglich ausgebildeten Haltevorrichtung, wobei die Haltevorrichtung von einem Schubschlauch umgeben ist, derart, dass ein distales Ende des Schubschlauchs im Bereich des Endes des Katheters zu liegen kommt, wobei der Schubschlauch mittels eines beweglichen Schubelements koaxial zur Haltevorrichtung verschiebbar ist, so dass bei einer Verschiebung des Schubschlauchs in distaler Richtung das Ende des Katheters von dem distalen Ende der Haltevorrichtung gelöst wird und das Schubelement, das Halteelement und der Schubschlauch zumindest teilweise innerhalb eines Gehäuses angeordnet sind, wobei das Gehäuse mindestens eine distale Öffnung für den Austritt des Halteelements und/oder des Schubschlauchs aufweist.

Derartige Platziervorrichtungen für Katheter sind zum Beispiel aus der DE 201 13 815 U bekannt.

Insbesondere dienen Platziervorrichtungen zur Platzierung und Steuerung eines Katheters während der Einführung und Platzierung in entsprechenden Körperöffnungen und Körpergefäßen. Dabei wird die üblicherweise schlauchartige Haltevorrichtung an dem der Körperöffnung gegenüberliegenden Seite des einzuführenden Katheters lösbar befestigt. Über diese Art Verlängerung wird das Einführen und das Platzieren des Katheters innerhalb der Körperöffnung oder des Körpergefäßes gesteuert. Nach der Platzierung des Katheters an der gewünschten Stelle innerhalb des Körpers muss die Haltevorrichtung vom Katheter gelöst werden. Dies erfolgt unter anderem manuell, einerseits durch ein ruckartiges Zurückziehen der Haltevorrichtung, so dass sich die Haltevorrichtung vom Katheterende löst. Andererseits sind sogenannte Dekonnektoren bekannt, die koaxial zur Haltvorrichtung verlaufen und über dieser geführt sind und an der Verbindungsstelle zwischen dem Katheter und der Haltevorrichtung angreifen und bei einer entsprechenden Krafteinwirkung diese voneinander lösen.

Nachteilig an den bekannten Platziervorrichtungen ist jedoch, dass durch das ruckartige und/oder manuelle Lösen der Haltevorrichtung von dem Katheter, insbesondere auch durch die bekannten Dekonnektriervorrichtungen, es sehr häufig zu Verletzungen innerhalb der Körperöffnungen und Körpergefäße kommt. Das Platzieren des Katheters mit derartigen bekannten Platziervorrichtungen kann daher nur sehr langsam und vorsichtig durchgeführt werden.

Es ist daher Aufgabe der vorliegenden Erfindung eine Platziervorrichtung der eingangs genannten Art bereitzustellen, welche einerseits ein leichtes Lösen des Katheters von der Platziervorrichtung und andererseits ein ruckfreies Verschieben eines Schubschlauches und damit ein ruckfreies Lösen der Platziervorrichtung von dem Katheter ermöglicht.

Gelöst wird diese Aufgabe durch eine Platziervorrichtung mit den Merkmalen des Anspruchs 1. Vorteilhafte Ausgestaltungen sind in den Unteransprüchen beschrieben.

Bei einer erfindungsgemäßen Platziervorrichtung sind ein Schubelement, ein Halteelement und ein Schubschlauch zumindest teilweise innerhalb eines Gehäuses angeordnet, wobei das Gehäuse mindestens eine distale Öffnung für den Austritt des Halteelementes und/oder des Schubschlauches aufweist. Durch die Anordnung der genannten Elemente der Platziervorrichtung innerhalb eines Gehäuses entsteht eine sehr kompakte Konstruktion, die sehr leicht handhabbar ist. Zudem bietet das Gehäuse Schutz vor möglichen Beschädigungen der genannten Elemente. Dabei besteht das Gehäuse insbesondere aus einem Gehäuseoberteil und einem Gehäuseunterteil, wobei das Gehäuseoberteil und das Gehäuseunterteil zueinander drehbar ausgebildet sind. Vorteilhafterweise ist an einem Innenumfang des Gehäuseoberteils mindestens eine Führung zur Führung von mindestens einem an dem Schubelement ausgebildeten Rastelement angeordnet, wobei die Führung mindestens jeweils zwei Rastausnehmungen zur Aufnahme des Rastelementes aufweist und die Rastausnehmungen in distaler Richtung zueinander versetzt ausgebildet sind. Eine derartige vorteilhafte Ausgestaltung der Platziervorrichtung erlaubt es, durch ein einfaches Drehen des Gehäuseoberteils relativ zum Gehäuseunterteil das Schubelement parallel zum Gehäuse bzw. parallel zum Halteelement innerhalb des Gehäuses zu verschieben, so dass es zu einer Verschiebung des Schubschlauches kommt und das entsprechende Ende des Katheters von dem distalen Ende der Haltvorrichtung gelöst wird. Insbesondere gewährleistet diese erfindungsgemäße Konstruktion ein ruckfreies Verschieben des Schubschlauches und damit ein ruckfreies Lösen der Platziervorrichtung von dem Katheter. Zudem erfordert diese Konstruktion nur einen minimalen Kraftaufwand für das Lösen des Katheters von der Platziervorrichtung. Zudem ist die länglich ausgebildete Haltevorrichtung von dem Schubschlauch umgeben ist, derart, dass ein distales Ende des Schubschlauchs im Bereich eines Endes des Katheters zu liegen kommt und der Schubschlauch mittels eines beweglichen Schubelements koaxial zur Haltevorrichtung verschiebbar ist, so dass bei einer Verschiebung des Schubschlauchs in distaler Richtung das Ende des Katheters von dem distalen Ende der Haltvorrichtung gelöst wird. Durch eine derartige Konstruktion ist ein leichtes Lösen des Katheters von der Platziervorrichtung möglich, da das Lösen über eine Verschiebung des Schubschlauches erfolgt, der wiederum mittels des beweglichen Schubelementes bewegt wird. Das distale Ende des Schubschlauches überträgt dabei eine entsprechende Kraft des beweglichen Schubelementes auf das Ende des Katheters, so dass dieser von der Haltevorrichtung gelöst wird. Vorteilhafterweise erfolgt diese Kraftübertragung ohne ruckartige Bewegungen, so dass die Gefahr einer Verletzung der Körperöffnungen oder Körpergefäße durch ein Lösen der Platziervorrichtung von dem im Körper verbleibenden Katheter minimiert wird.

In einer vorteilhaften Ausgestaltung der Erfindung ist die Haltevorrichtung schlauch- oder rohrartig oder massiv ausgebildet. Insbesondere kann die Haltevorrichtung schlauch- oder rohrartig ausgebildet sein, so dass innerhalb der Haltevorrichtung koaxial ein Führungsdraht platziert werden kann.

In einer weiteren vorteilhaften Ausgestaltung der Erfindung ist der Schubschlauch elastisch oder fest ausgebildet. Je nach Anforderungen, das heißt je nach Anwendungsgebiet des Katheters kann der Schubschlauch der Platziervorrichtung entsprechende Materialeigenschaften aufweisen. Ein elastischer Schubschlauch eignet sich insbesondere für sehr lang ausgebildete Platziervorrichtungen, ein fest ausgebildeter Schubschlauch kann insbesondere dann verwendet werden, wenn große Kräfte zum Lösen der Platziervorrichtung von dem Katheter notwendig sind.

In einer weiteren vorteilhaften Ausgestaltung der Erfindung ist in einem distalen Bereich des Gehäuseunterteils ein Federelement angeordnet, wobei das Federelement den Schubschlauch umgibt und an dem einem distalen Bereich des Gehäuseunterteils gegenüberliegenden Ende an einem Verlängerungselement des Schubelementes anliegt. Die durch das Federelement ausgeübte Kraft gewährleistet einen gleichmäßigen Kraftverlauf und eine entsprechende gleichmäßige Kraftausübung des Schubelementes auf den Schubschlauch. Des weiteren ermöglicht das Federelement eine automatische Rückführung des Schubschlauchs in seine Ausgangsposition.

In einer weiteren vorteilhaften Ausgestaltung der Erfindung ist das Gehäuseunterteil im distalen Bereich verjüngt ausgebildet. Eine derartige Ausgestaltung des Gehäuseunterteils vereinfacht die Handhabung der Platziervorrichtung deutlich.

In einer weiteren vorteilhaften Ausgestaltung der Erfindung weist das Gehäuse an dem dem distalen Bereich des Gehäuseunterteils gegenüberliegenden Ende eine Adaptervorrichtung zum Anschluss weiterer medizinischer und chirurgischer Vorrichtungen auf. Damit ist gewährleistet, dass die erfindungsgemäße Platziervorrichtung einen sehr großen Anwendungsbereich aufweist.

Weitere Einzelheiten, Merkmale und Vorteile der Erfindung ergeben sich aus der nachfolgenden Beschreibung eines in den Figuren dargestellten Ausführungsbeispiels. Es zeigen
- Figur 1: eine schematische Darstellung der erfindungsgemäßen Platziervorrichtung mit einem aufgesetztem Katheter; und
- Figur 2: eine schematische Darstellung der erfindungsgemäßen Platziervorrichtung mit gelöstem Katheter.

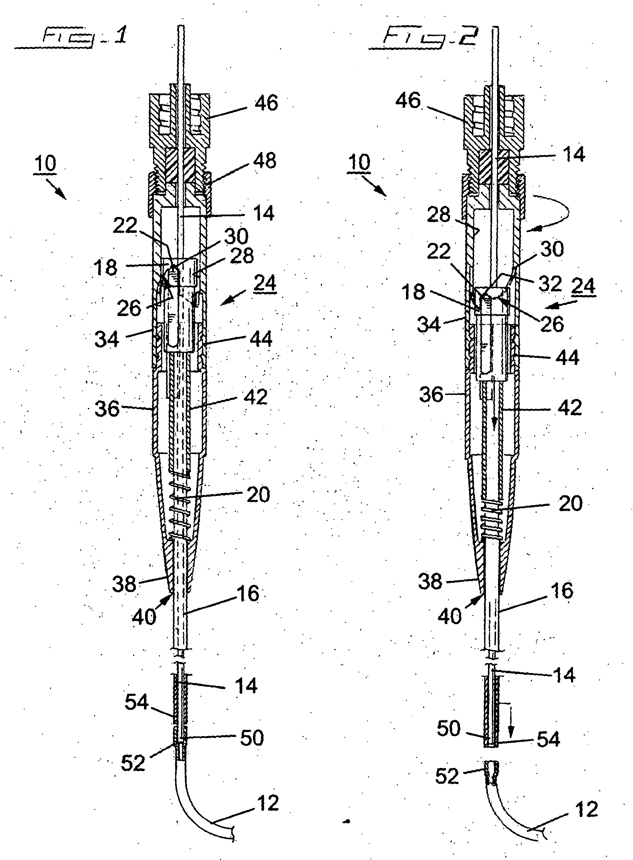

Figur 1 zeigt in einer schematischen Darstellung eine Platziervorrichtung 10 mit einem aufgesetzten Katheter 12. Man erkennt, dass der Katheter 12 mit einem Ende lösbar mit einer länglich ausgebildeten Haltevorrichtung 14 verbunden ist.

Insbesondere ist ein distales Ende 50 der Haltevorrichtung 14 in ein Ende 52 des Katheters 12 eingeführt.

Die Haltevorrichtung 14 ist von einem Schubschlauch 16 koaxial umgeben, derart, dass ein distales Ende 54 des Schubschlauchs 16 im Bereich des Endes 52 des Katheters 12 zu liegen kommt oder an diesem anliegt. Dabei ist der Schubschlauch 16 mit einem Schubelement 18 verbunden, wobei das Schubelement 18 koaxial zur Haltevorrichtung 14 verschiebbar ist. Damit ergibt sich auch eine koaxiale Verschiebbarkeit des Schubschlauches 16 gegenüber der Haltevorrichtung 14, die in dem Ausführungsbeispiel ebenfalls schlauchartig ausgebildet ist. Man erkennt, dass bei einer Verschiebung des Schubschlauches 16 in distaler Richtung das Ende 52 des Katheters 12 von dem distalen Ende 50 der Haltevorrichtung 14 gelöst wird (vergleiche Figur 2).

Des weiteren erkennt man, dass das Schubelement 18, die Haltevorrichtung 14 und der Schubschlauch 16 zumindest teilweise innerhalb eines Gehäuses 24 angeordnet bzw. geführt sind.Das Gehäuse 24 weist eine distale Öffnung 40 für den Austritt der Haltevorrichtung 14 und des Schubschlauchs 16 auf. Das Gehäuse 24 besteht dabei aus einem Gehäuseoberteil 34 und einem Gehäuseunterteil 36. Das Gehäuseoberteil 34 und das Gehäuseunterteil 36 sind dabei über eine Drehverbindung 44 zueinander drehbar ausgebildet. Zudem erkennt man, dass an einem Innenumfang 28 des Gehäuseoberteils 34 eine umgehende Führung 26 zur Führung von zwei an dem Schubelement 18 ausgebildeten Rastelementen 22 angeordnet ist. Die Führung 26 weist dabei für jedes Rastelement 22 zwei Rastausnehmungen 30, 32 zur Aufnahme des Rastelementes 22 auf. Die Rastausnehmungen 30, 32 sind dabei in distaler Richtung zueinander versetzt ausgebildet. Die beiden Rastausnehmungen 30, 32 bilden somit die Endpunkte für die Führung der Rastelemente 22. Durch das Drehen des Gehäuseoberteils 34 relativ zum Gehäuseunterteil 36 werden die Rastnasen 22 entlang der Führung 26 geführt, so dass es zu einer koaxialen Verschiebung des Schubelementes 18 innerhalb des Gehäuses 24 kommt. Diese Verschiebung des Schubelementes 18 bewirkt dann eine entsprechende Verschiebung bzw. Bewegung des Schubschlauchs 16, wie aus Figur 2 deutlich erkennbar ist. Die Verschiebung des Schubelementes 16 führt dann zum gewünschten Lösen des Katheters 12 von der Platziervorrichtung 10. In der dargestellten Ausführungsform weist die Führung 26 zwei Rastausnehmungen 30, 32 auf. Es ist aber auch möglich weitere Rastausnehmungen innerhalb der Führung 26 auszubilden.

Des weiteren erkennt man, dass in einem distalen Bereich 38 des Gehäuseunterteils 36 ein Federelement 20 angeordnet ist. Das Federelement 20 umgibt dabei den Schubschlauch 16. In dem dem distalen Bereich 38 des Gehäuseunterteils 36 gegenüberliegenden Ende liegt das Federelement 20 an einem Verlängerungselement 42 des Schubelements 18 an. Das Federelement 20 garantiert einen ruckfreien Vorschub des Schubelementes 18 innerhalb des Gehäuses 24 und somit einen ebenfalls ruckfreien Vorschub des Schubschlauches 16. Zudem ist es möglich, den Schubschlauch 16 bzw. das Schubelement 18 in die entsprechende Ausgangsposition wieder zurückzuführen. Schließlich erkennt man, dass das Gehäuseunterteil 36 im distalen Bereich 38 verjüngt ausgebildet ist. Dadurch ist das Gehäuse 24 insgesamt stiftartig ausgebildet, so dass die Platziervorrichtung 10 einfach gehandhabt werden kann.

Des weiteren erkennt man, dass das Gehäuse 24 an dem dem distalen Bereich 38 des Gehäuseunterteils 36 gegenüberliegenden Ende über ein lösbares Verbindungselement 48 eine Adaptervorrichtung 46 zum Anschluss weiterer medizinischer oder chirurgischer Vorrichtungen aufweist.

Die beschriebene Haltevorrichtung 14 kann schlauch- oder rohrartig oder massiv ausgebildet sein. In dem dargestellten Ausführungsbeispiel ist die Haltevorrichtung 14 schlauchartig ausgebildet, wobei innerhalb der Haltevorrichtung 14 ein Führungsdraht geführt werden kann. Der Schubschlauch 16 kann elastisch oder fest ausgebildet sein. Beide Elemente, nämlich die Haltevorrichtung 14 und der Schubschlauch 18 bestehen aus einem bioverträglichen und biostabilen Material. Hierfür stehen unter anderem einen Vielzahl von Kunststoffen zur Verfügung.

## Patentansprüche

1. Platziervorrichtung (10) für einen Katheter (12) zum Einführen des Katheters (12) in Körperöffnungen und Körpergefäße bestehend aus einer an einem Ende (52) des Katheters (12) lösbar mit dem Katheter (12) verbindbaren länglich ausgebildeten Haltevorrichtung (14), wobei die Haltevorrichtung (14) von einem Schubschlauch (16) umgeben ist, derart, dass im Gebrauch ein distales Ende (54) des Schubschlauchs (16) im Bereich des Endes (52) des Katheters (12) zu liegen kommt, wobei der Schubschlauch (16) mittels eines beweglichen Schubelements (18) koaxial zur Haltevorrichtung (14) derart verschiebbar ist, dass bei einer Verschiebung des Schubschlauchs (16) in distaler Richtung das Ende (52) des Katheters (12) von dem distalen Ende (50) der Haltevorrichtung (14) lösbar ist und das Schubelement (18), die Haltevorrichtung (14) und der Schubschlauch (16) zumindest teilweise innerhalb eines Gehäuses (24) angeordnet sind, wobei das Gehäuse (24) mindestens eine distale Öffnung (40) für den Austritt der Haltevorrichtung (14) und/oder des Schubschlauchs (16) aufweist,
**dadurch gekennzeichnet,**
**dass** das Gehäuse (24) aus einem Gehäuseoberteil (34) und einem Gehäuseunterteil (36) besteht, wobei das Gehäuseoberteil (34) und das Gehäuseunterteil (36) zueinander drehbar ausgebildet sind und an einem Innenumfang (28) des Gehäuseoberteils (34) mindestens eine Führung (26) zur Führung von mindestens einem an dem Schubelement (18) ausgebildeten Rastelement (22) angeordnet ist, wobei die Führung (26) mindestens jeweils zwei Rastausnehmungen (30, 32) zur Aufnahme des Rastelements (22) aufweist und die Rastausnehmungen (30, 32) in distaler Richtung zueinander versetzt ausgebildet sind.

2. Platziervorrichtung nach Anspruch 1,
**dadurch gekennzeichnet,**
**dass** die Haltevorrichtung (14) schlauch- oder rohrartig oder massiv ausgebildet ist.

3. Platziervorrichtung nach Anspruch 1 oder 2,
**dadurch gekennzeichnet,**
**dass** der Schubschlauch (16) elastisch oder fest ausgebildet ist.

4. Platziervorrichtung nach Anspruch 1,
**dadurch gekennzeichnet,**
**dass** in einem distalen Bereich (38) des Gehäuseunterteils (36) ein Federelement (20) angeordnet ist und das Federelement (20) den Schubschlauch (16) umgibt, wobei das Federelement (20) an dem dem distalen Bereich (38) gegenüberliegenden Ende an einem Verlängerungselement (42) des Schubelements (18) anliegt.

5. Platziervorrichtung nach einem der Ansprüche 1 oder 4,
**dadurch gekennzeichnet,**
**dass** das Gehäuseunterteil (36) im distalen Bereich (38) verjüngt ausgebildet ist.

6. Platziervorrichtung nach einem der Ansprüche 1 oder 4 oder 5,
**dadurch gekennzeichnet,**
**dass** das Gehäuse (24) an dem dem distalen Bereich (38) des Gehäuseunterteils (36) gegenüberliegenden Ende eine Adaptervorrichtung (46) zum Anschluss weiterer medizinischer oder chirurgischer Vorrichtungen aufweist.

7. Platziervorrichtung nach einem der vorhergehenden Ansprüche,
**dadurch gekennzeichnet,**
**dass** das Halteelement (14) und der Schubschlauch (16) aus einem bioverträglichen und biostabilen Material bestehen.

## Claims

1. Placement device (10) for a catheter (12) for introducing the catheter (12) into body openings and body vessels, consisting of an elongated formed retaining device (14) detachably connectable to the catheter (12) at and end (52) of the catheter (12), wherein the retaining device (14) is surrounded by a push hose (16) such that a distal end (54) of the push hose (16) comes to lie in the region of the end (52) of the catheter (12) in use, wherein the push hose (16) is displaceable coaxially to the retaining device (14) by means of a movable push member (18) such that upon displacement of the push hose (16) in the distal direction, the end (52) of the catheter (12) is detachable from the distal end (50) of the retaining device (14), and the push member (18), the retaining device (14) and the push hose (16) are disposed at least partially within a housing (24), wherein the housing (24) has at least one distal opening (40) for the exit of the retaining device (14) and/or the push hose (16),
**characterized in that**
the housing (24) is comprised of a housing top (34) and a housing bottom (36), wherein the housing top (34) and the housing bottom (36) are formed rotatably with respect to each other, and at least one guide (26) for guiding at least one locking member (22) formed on the push member (18) is disposed on an inner circumference (28) of the housing top (34), wherein the guide (26) has at least each two locking recesses (30, 32) for receiving the locking member (22), and the locking recesses (30, 32) are formed offset from each other in the distal direction.

2. Placement device according to claim 1,
**characterized in that**
the retaining device (14) is formed hose- or tube-like or solidly.

3. Placement device according to claim 1 or 2,
**characterized in that**
the push hose (16) is formed resiliently or rigidly.

4. Placement device according to claim 1,
**characterized in that**
a spring member (20) is disposed in a distal region (38) of the housing bottom (36), and the spring member (20) surrounds the push hose (16), wherein the spring member (20) abuts an extension member (42) of the push member (18) at the end opposing the distal region (38).

5. Placement device according to any one of claims 1 or 4,
**characterized in that**
the housing bottom (36) is formed tapered in the distal region (38).

6. Placement device according to any one of claims 1 or 4 or 5,
**characterized in that**
the housing (24) has an adapter device (46) for connecting further medical or surgical devices, at the end opposing the distal region (38) of the housing bottom (36).

7. Placement device according to any one of the preceding claims,
**characterized in that**
the retaining member (14) and the push hose (16) are made of a biocompatible and biostable material.

## Revendications

1. Dispositif de positionnement (10) d'un cathéter (12) pour l'introduction du cathéter (12) dans des ouvertures corporelles et des vaisseaux corporels, qui se constitue d'un dispositif de maintien (14) de forme longitudinale pouvant être relié de façon amovible au cathéter (12 en une extrémité (52) du cathéter (12), le dispositif de maintien (14) étant entouré d'un tuyau de poussée (16), de manière telle qu'à l'utilisation, une extrémité distale (54) du tuyau de poussée (16) vienne reposer dans la zone de l'extrémité (52) du cathéter (12), le tuyau de poussée (16) pouvant être glissé coaxialement au dispositif de maintien (14) au moyen d'un élément mobile de poussée (18), que lors d'un glissement du tuyau de poussée (16) en direction distale, l'extrémité (52) du cathéter (12) peut être séparée de l'extrémité distale (50) du dispositif de maintien (14) et que l'élément de poussée (18), le dispositif de maintien (14) et le tuyau de poussée (16) sont disposés au moins partiellement dans un boîtier (24), le boîtier (24) présentant au moins une ouverture distale (40) pour la sortie du dispositif de maintien (14) et/ou du tuyau de poussée (16),
**caractérisé en ce que**
le boîtier (24) se constitue d'une partie supérieure de boîtier (34) et d'une partie inférieure de boîtier (36), la partie supérieure de boîtier (34) et la partie inférieure de boîtier (36) étant réalisées de façon à pouvoir pivoter l'une par rapport à l'autre, et qu'en une périphérie intérieure (28) de la partie supérieure de boîtier (34), est disposé au moins un guidage (26) pour guider au moins un élément d'engagement (22) formé sur l'élément de poussée (18), le guidage (26) présentant chaque fois au moins deux évidements d'engagement (30, 32) pour recevoir l'élément d'engagement (22) et les évidements d'engagement (30, 32) étant constitués pour être décalés l'un par rapport à l'autre en direction distale.

2. Dispositif de positionnement selon la revendication 1,
**caractérisé en ce que**
le dispositif de maintien (14) est constitué comme un tuyau ou un tube ou est plein.

3. Dispositif de positionnement selon la revendication 1 ou 2,
**caractérisé en ce que**
le tuyau de poussée (16) est élastique ou dur.

4. Dispositif de positionnement selon la revendication 1,
**caractérisé en ce que**
qu'en une zone distale (38) de la partie inférieure de boîtier (36) est disposé un élément élastique (20) et que l'élément élastique (20) entoure le tuyau de poussée (16), l'élément élastique (20) reposant contre un élément de prolongement (42) de l'élément de poussée (18) à l'extrémité située en face de la zone distale (38).

5. Dispositif de positionnement selon l'une quelconque des revendications 1 ou 4,
**caractérisé en ce que**
la partie inférieure de boîtier (36) s'amincit dans la zone distale (38).

6. Dispositif de positionnement selon l'une quelconque des revendications 1 ou 4 ou 5,
**caractérisé en ce que**
le boîtier (24) présente, à l'extrémité située en face de la zone distale (38) de la partie inférieure de boîtier (36), un dispositif d'adaptation (46) pour raccorder d'autres dispositifs médicaux ou chirurgicaux.

7. Dispositif de positionnement selon l'une quelconque des revendications précédentes,
**caractérisé en ce que**
l'élément de maintien (14) et le tuyau de poussée (16) sont constitués en un matériau biocompatible et biostable.
